# EUROPEAN PATENT SPECIFICATION

(11) **EP 1 169 968 B1**
(45) Date of publication and mention of the grant of the patent: **30.01.2002**
(21) Application number: 00850184.3
(22) Date of filing: 03.11.2000
(51) Int. Cl.: A61B 17/00

(54) **Sealing and wound closure device**
Vorrichtung zum Abdichten und Verschliessen von Wunden
Dispositif de scellage et occlusion pour blessures

(43) Date of publication of application: 09.01.2002
(73) Proprietor: RADI MEDICAL SYSTEMS AB, 754 50 Uppsala (SE)
(72) Inventor: Akerfeldt, Dan, 755 92 Uppsala (SE); Preiniz, Fredrik, 754 48 Uppsala (SE); Egnelöv, Per, 754 40 Uppsala (SE)
(74) Representative: Hytting, Kerstin Cecilia

(56) References cited:
- WO-A-99/40849
- US-A- 5 531 759
- US-A- 5 620 461

## Description

### FIELD OF THE INVENTION

The present invention relates to a sealing device and a wound closure device according to the preamble of claims 1, 6 and 7, respectively.

### BACKGROUND

During certain types of medical surgery or treatment an introducer is used to access the vascular system of a patient. The introducer is inserted through the wall of a blood vessel in order to obtain access to the vascular system and may thereafter be used for guiding medical instruments such as catheters, guide wires and the like.
After the completion of the medical procedure there will be an incision or a wound in the wall of the blood vessel corresponding to the size of the introducer. The bleeding from the wound, which is the result of such a surgical operation, may be stopped by applying direct pressure on the wound. However, applying direct pressure on the wound will require assistance of medical personnel and may also restrict the blood flow through the vessel.

US 5,342,393 describes a sealing device for sealing a perforation in the wall of a blood vessel. The device is in the form a two-part rivet, which seals the hole from both the inside and the outside of the vessel. Two rivet portions, an outer rivet portion and an inner rivet portion, are provided which are bayonet or screw threadedly interlocked to clamp the vessel wall. A problem with such a sealing device is that when screwing the outer rivet portion also the inner rivet portion will rotate since there is no holding-up on the inner rivet portion from the inside of the vessel, and thus it is hard to know if the sealing device is properly sealed or not. The latter may have disastrous consequences with blood leaking vessels.

In US 5,342,393 is also shown a sealing device, locked together via interlocking ridges. The inner rivet stem has triangular ridges that define horizontal shoulders and the outer rivet may have one or more upside down triangular ridges that define an opposing shoulder for engaging an adjacent shoulder and to thereby allow the outer rivet to be slid over the inner rivet but prevent separating movement. So in this sealing device, both the inner rivet stem and the outer rivet HAVE so-called saw teeth.
A problem with this sealing device is that the distance between the two occluders (i.e. rivets) may be varied only between fixed positions and thus cannot be exactly adapted to the thickness of the vessel. This in turn can lead to deteriorated tightening of the vessel if the distance exceeds the thickness of the vessel. On the other hand, it can also lead to the vessel being clamped with undue pressure on the vessel, which in turn might result in tissue necrosis or rupture of the arterial wall, when the distance is less than the thickness of the vessel.

The closest prior art is shown in US 5,350,399 which forms the basis for the preamble of claim 1. This document shows another sealing device for sealing a perforation in the wall of a blood vessel. The sealing device is constructed of a first member in the form of an intra-arterial occluder comprising a guide means in the form of an elongated wire integral with and extending centrally from the intra-arterial occluder and a second member in the form of an extra-arterial occluder. The guide includes a portion extending from the intra-arterial occluder which contains a plurality of saw teeth while the extra-arterial occluder is provided with an opening through which the guide passes. The saw teeth are wider in diameter than the opening in the extra-arterial occluder so that the extra-arterial occluder can be passed over the teeth in a direction towards the intra-arterial occluder.
The above mentioned problem, with the distance between the two occluders being varied only between fixed positions and thus cannot be exactly adapted to the thickness of the vessel, also arises in this sealing device.
A solution to the problem concerning saw teeth would be to provide very fine divided saw teeth, but this would be very unpractical and would give rise to manufacturing problems.

The inventors found that a sealing device commonly is inserted via an elongated introducer extending not perpendicular to the blood vessel, but in an acute angle towards the vessel, to get a insertion direction which is as close to the direction of the vessel as possible. This implies that the surfaces of the sealing device that is to be in contact with the surface of the vessel wall are not parallel to the surface of the vessel in the sealing moment, but in an acute angle to the surface of the vessel.
This reveals another problem in all the above mentioned sealing devices of prior art, if they have a rather stiff inner rivet stem portion extending centrally from the intra-arterial occluder. This problem concerns the difficulty of bringing the two occluders close enough to each other so as to tighten the vessel, because if the occluders cannot be adapted to the vessel wall, the vessel wall has to be deformed to fit towards the occluders which requires an increased sealing force between the occluders.
The angle also causes tensions to the elastic vessel wall since the vessel wall that is in contact with the sealing device becomes parallel to the surface of the occluders which makes the incision of the vessel wall dilated and the tightening more difficult.
The inventors found that these two problems do not arise at all when said sealing device surfaces are parallel to said vessel surface in the sealing moment, but the problem increases the larger the angle becomes.
The inventors found that if the inner rivet stem portion on the other hand is not stiff but flexible, the surface of the inner rivet portion will easy be adjusted to a position, which is parallel to the surface of the vessel. The stem closest to the inner rivet will be perpendicular to the inner rivet surface and the surface of the vessel, but be bent and making an arc, towards the acute angle of the elongated introducer where it thus is perpendicular to the surface of the outer rivet which is to be in contact with the surface of the vessel. The distance between the saw teeth on the stem will then be different on the outside and the inside of said arc, making it problematic to get a good engagement between the saw teeth on the stem and the outer rivet.

Thus there is a need in the art for a sealing device having improved and more secure sealing properties.

### SUMMARY OF THE INVENTION

The objective problem to be solved by the present invention is to provide a sealing device for closing a wound, which has enhanced tightening properties.

This problem is solved by the invention according to the features of the independent claims 1, 6 and 7.

Since the elongated member constitutes or is coated by a suture wire, and has a constant thickness along its lock portion, the thickness being greater than the opening of the second sealing member, the sealing device is infinitely variable lockable along the distal lock portion.

Preferred embodiments of the plug according to the invention are defined in the dependent claims 2-5.

Thus, it is believed that the present invention provides a novel and easy-to-use sealing and wound closure device for closing a wound with excellent sealing properties.

### BRIEF DESCRIPTION OF THE DRAWINGS

The foregoing and other aspects of the present invention will be best appreciated with reference to the following detailed description of specific embodiments of the invention, given by way of example only, when read in conjunction with the accompanying drawing, wherein

**Figure 1** shows a sealing device for closing a wound in a wall of a vessel according to a first embodiment of the invention.

**Figure 2** shows a sectional view of a first sealing member.

**Figure 3** shows a sectional view of a second sealing member.

**Figure 4** shows a sealing device for closing a wound in a wall of a vessel according to a first embodiment of the invention,

**Figure 5** shows an elongated core.

**Figure 6** shows a sealing device for closing a wound in a wall of a vessel according to a second embodiment of the invention.

**Figure 7** shows a sealing device for closing a wound in a wall of a vessel according to a third embodiment of the invention.

**Figure 8** shows a wound closer device, which comprises the sealing device of any of the embodiments.

**Figure 9** shows a sectional view of a wound site, with an introducer extending through the vesse] wall and into the vessel.

**Figure 10** shows a sectional view of the wound site, with the pusher, the first sealing member, the elongated member and the second sealing member, inside of the introducer.

**Figure 11** shows a sectional view of the wound site wherein the first sealing member extends outside of the introducer and into the vessel.

**Figure 12** shows a sectional view of the wound site wherein the introducer is drawn out of the vessel 30 and the second sealing member is pushed outside the introducer.

**Figure 13** shows a sectional view of the wound site wherein the second sealing member is pushed into its final position.

**Figure 14** shows a sectional view of the wound site wherein the the sealing device closes the wound and the pusher and the introducer are removed.

**Figure 15** shows a sealing device for closing a wound in a wall of a vessel according to a fifth embodiment of the invention.

**Figure 16** shows a sealing device for closing a wound in a wall of a vessel according to a fifth embodiment of the invention.

**Figure 17** shows a sealing device for closing a wound in a wall of a vessel according to a sixth embodiment of the invention.

**Figure 18** shows a cross sectional view of the first sealing member compressed inside the introducer according to Figure 8, seen along the line A-A.

### DESCRIPTION OF THE PREFERRED EMBODIMENTS

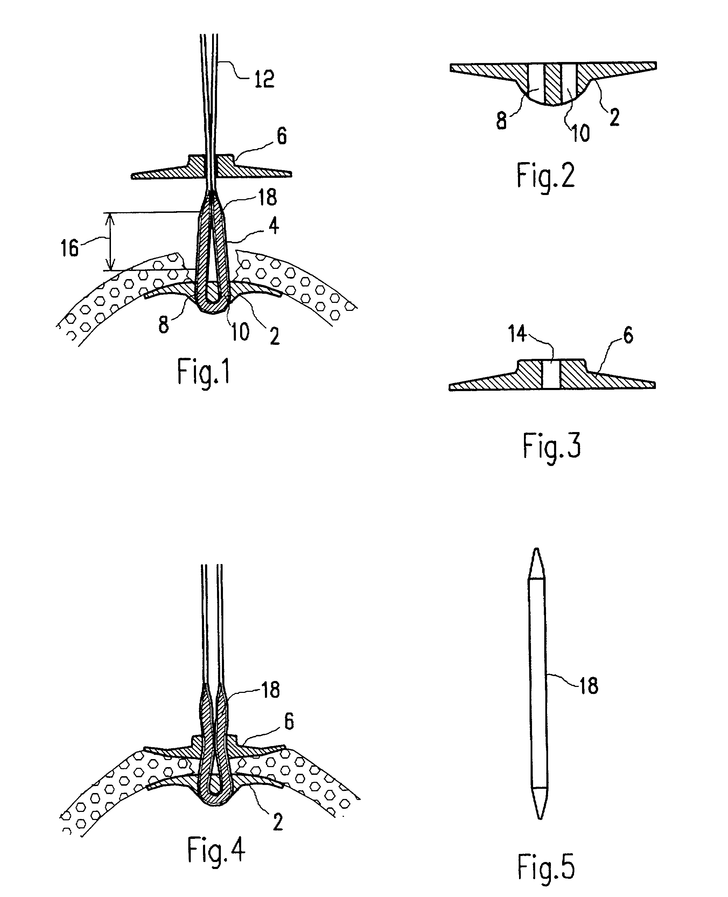

**Figure 1** shows a sealing device for closing a wound in a wall of a vessel according to a first embodiment of the invention. The sealing device comprises three separate parts, namely a first sealing member 2, an elongated member 4 and a second sealing member 6. The first sealing member 2 is attached to a distal end of the elongate member 4. In this first embodiment of the sealing device, the first sealing member comprises two through openings 8, 10 (**Figure 2**) through which a multifilament suture wire 12 is thread so as to make a pair of suture wires constituting the elongated member 4.

The second sealing member 6 is provided with an opening 14 (**Figure 3**), which is adapted to the elongate member 4, i.e. the opening 14 is greater than the thickness of the proximal portion of the elongate member 4. With a structure like this the second sealing member 6 is threadable onto and along the elongate element 4 (**Figure 1**). The most distal portion of the elongate member 4 has a constant thickness that is slightly greater than the opening 14 of the second sealing member 6 and constitutes the distal lock portion 16.

This will allow for frictional engagement between inside of the opening 14 of the second sealing member 6 and the distal lock portion 16 of the elongate member 4 which makes the sealing device infinitely variable lockable along said distal lock portion 16 (**Figure 4**).

The multifilament suture wire 12 is preferably made of a resorbable material such as Glycolic/Lactide polymer. The first sealing member 2 and second sealing member 6 are preferably made of a flexible resorbable material, such as Caprolactone/Trimethylene Carbonate/Glycolide polymer or any corresponding materials.

The choice of using a suture wire for the elongated element 4 is very important for the safe securing of the sealing device. Tests have been made to use the same material, e.g. a polymer, in the elongated member 4 as in the second sealing member 6. Since polymer gives a very glossy surface, it is hard to get high power frictional engagement between the elongated member 4 and the sealing member 6. Using a suture wire 12 for the elongate member 4 gives a safer sealing since the suture wire comprises a number of circulating fibres thus giving the wire a rough surface with a high frictional sealing power towards a glossy surface inside the opening 14 of the second sealing member 6.
The suture wire also makes the sealing device safer in another way. The suture wire is made in one piece and has very high tensile strength. It constitutes a continuous wire from the inner seal through the outer seal and to a tampering grip of the insertion tool, being threaded in through the first opening 8 and out again through the second opening 10 and thus keeping the sealing device safe together.
If a first sealing member and an elongated member are cast in one piece there is often problems with the casting process, giving the casted member air bubbles and inclusions and accordingly giving the sealing device poor structural strength.
The challenge is to make the suture wire 12 thicker in the distal lock portion 16.

In the first embodiment of the present invention, a hollow core of the suture wire 12 is filled with an elongated core 18 (**Figure 5**), within the area of the distal lock portion 16 of the elongate member 4, but also in the area which is to be threaded through the first sealing member 2. (See again **Figure 1**). The elongated core 18 is preferably made of a resorbable Caprolactone/Trimethylene carbonate/Glycolide polymer. This gives the suture wire 12 a thickening in the distal lock portion 16.

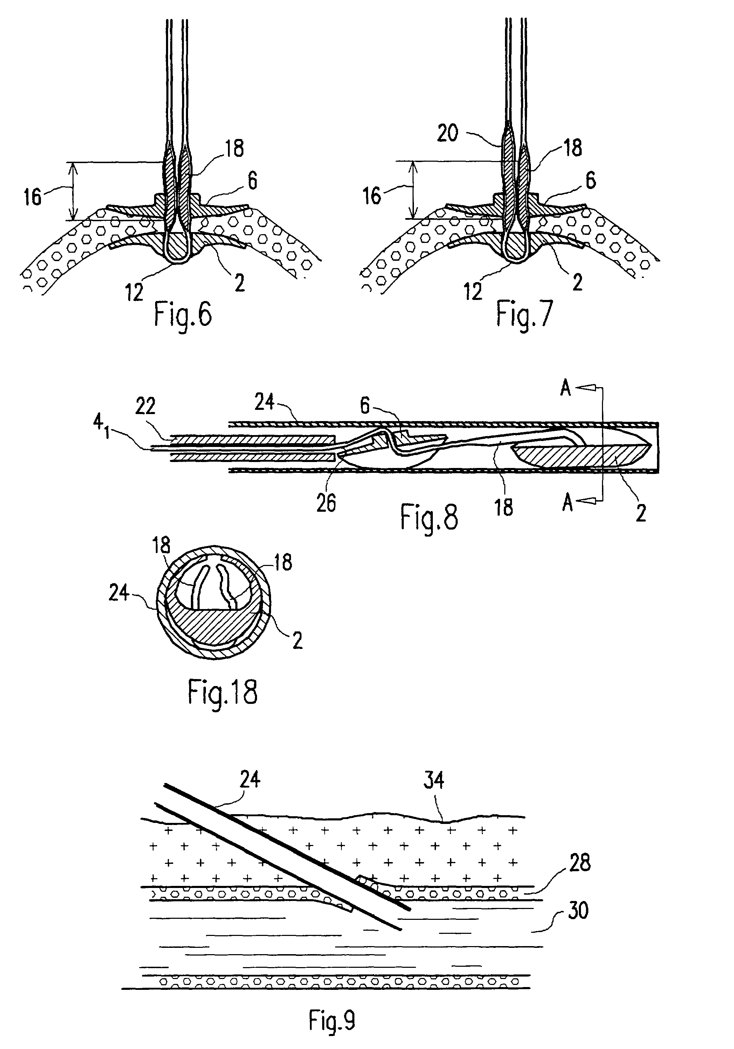

In a second embodiment of the present invention, shown in **Figure 6,** the suture wire 12 is left unfilled within the area ranging from the entry of the first opening 8 of the first sealing member 2, through the first sealing member 2, out on the other side and in again through the second opening 10 of the first sealing member 2 to the exit of said second opening 10.

In a third embodiment of the present invention, shown in **Figure 7**, the thickening of the first suture, of the two sutures making a pair of sutures, extends beyond the distal lock portion 16 into the proximal portion of the elongated member 4. This gives the suture wire 12 a more continuous increasing of the thickness which simplifies the threadening of the second sealing member 6 from the proximal portion onto the distal lock portion 16.

In a forth embodiment of the present invention, instead of being filled, the suture 12 is thicker woven in the area of the distal lock portion.

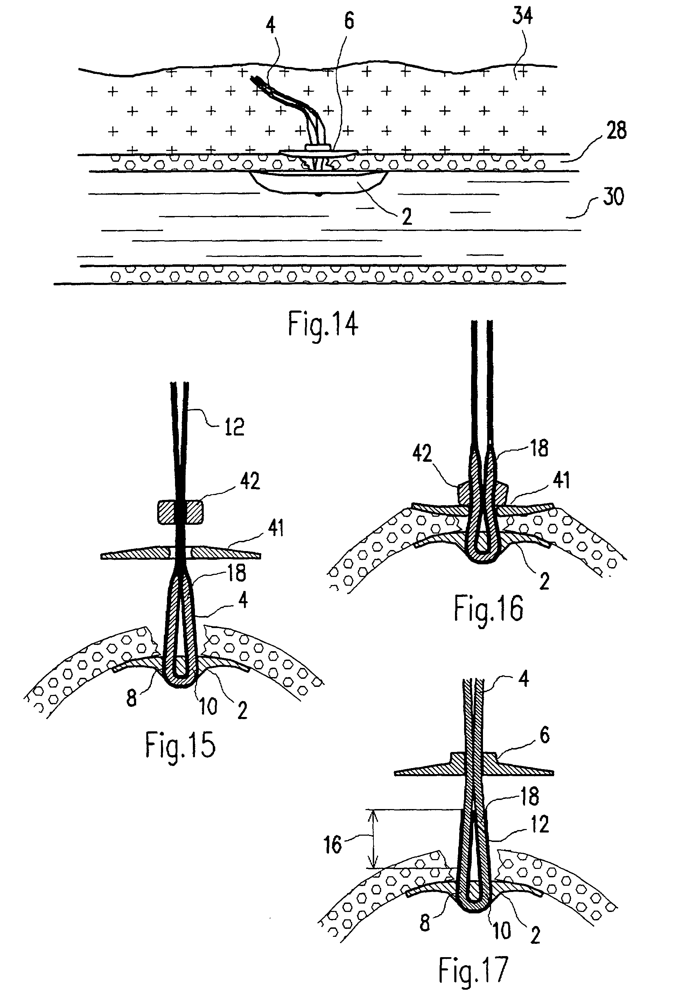

In a fifth embodiment of the present invention, (**Figure 15 and 16**) the second sealing member is divided into two parts, which first part 41 is a plate and is provided with an opening that is approximately the same or slightly greater than the thickness of the distal lock portion 16. This first part 41 is threadable onto and along the elongate member 4 (**Figure 15**), over the distal lock portion until it is in contact with the outside of the vessel wall. The first part plate 41 is preferably quite thin, which makes it flexible and easy to adapt to the vessel wall. The second part 42 is provided with an opening that is slightly smaller than the thickness of the distal lock portion 16. This second part 42 is threadable onto and along the elongate member 4 (**Figure 15**), over the distal lock portion until it is in contact with the first part 41. The second part 42 allows for frictional engagement between the inside of the opening of the second part 42 and the distal portion 16 (**Figure 16**). The second part 42 is preferably thicker than the first part 41, which gives it a large surface inside its opening for said frictional engagement. On the other hand, the diameter of the second part 42 is preferably smaller than the first part 41.

In a sixth embodiment, the elongated portion 4 is not a suture wire, but another material, e.g. a resorbable polymer (**Figure 17**). The distal lock portion 16 is coated by a hollow suture wire like a stocking so that a decent frictional engagement can be achieved between said coated distal lock portion and the inside of the opening of the second sealing member.

Typical dimensions of parts of the sealing device are for the first and second openings 8 and 10 of the first sealing member 2, a diameter of 0.2 mm, and for the opening 14 of the second sealing member 6, a diameter of 0.5 mm. The typical dimension of the suture is a diameter of 0.25 mm and of the distal lock portion 16, a diameter of 0.4 mm and a length of is 8 mm.

**Figure 2 and Figure 3** show respectively a sectional view of an example of a first sealing member 2 and a second sealing member 6 according to the embodiments of the invention. The first and second sealing member 2, 6 will pass through an introducer 24 (see **Figure 8**) on the way to its final position in the wound. Since the first and second sealing member 2, 6, each has a diameter that is greater than the diameter of the introducer 24, they are made compressible. The first and second sealing member 2 and 6, according to the present invention are preferably built up of portions that are foldable and portions that are more rigid, but other shapes are also possible. **Figure 18** shows a cross sectional view of the first sealing member 2 compressed inside the introducer 24 according to Figure 8, seen along the line A-A.

**Figure 8** shows a wound closer device, which comprises the sealing device of any of the embodiments, mentioned above and a pusher 22 adapted for pushing the first sealing member 2, the elongated member 4 and the second sealing member 6 through an introducer 24.
The sealing device will be passed through the introducer 24 in order to reach its final position in the wound to be closed. To achieve this, the pusher 22 may be used to push the first sealing member 2, the elongated member 4 and the second sealing member 6 through the introducer 24. The pusher 22 has a size adapted to the size of the introducer 24, i.e. the diameter of pusher 22 is smaller than the inner diameter of the introducer 24 such that the elements of the sealing device can be pushed through the introducer 24. Furthermore, the pusher 22 is provided with a through hole 26 along the longitudinal axis thereof. This through hole 26 is large enough to accommodate the elongate member 4.

It will now be described how the different elements of the wound closure device operate and their relation to each other.

**Figure 9** shows a sectional view of a wound site, with an introducer 24 extending through the vessel wall 28 and into the vessel 30. The introducer 24 is introduced through the skin and passes the tissue 34 before it penetrates the vessel wall 28 and enters the vessel 30. When the introducer 24 is removed a wound will be left in the vessel wall 28. It is that wound that the wound closer device according to the present invention will close, with use of the wound closure device and sealing device described above.

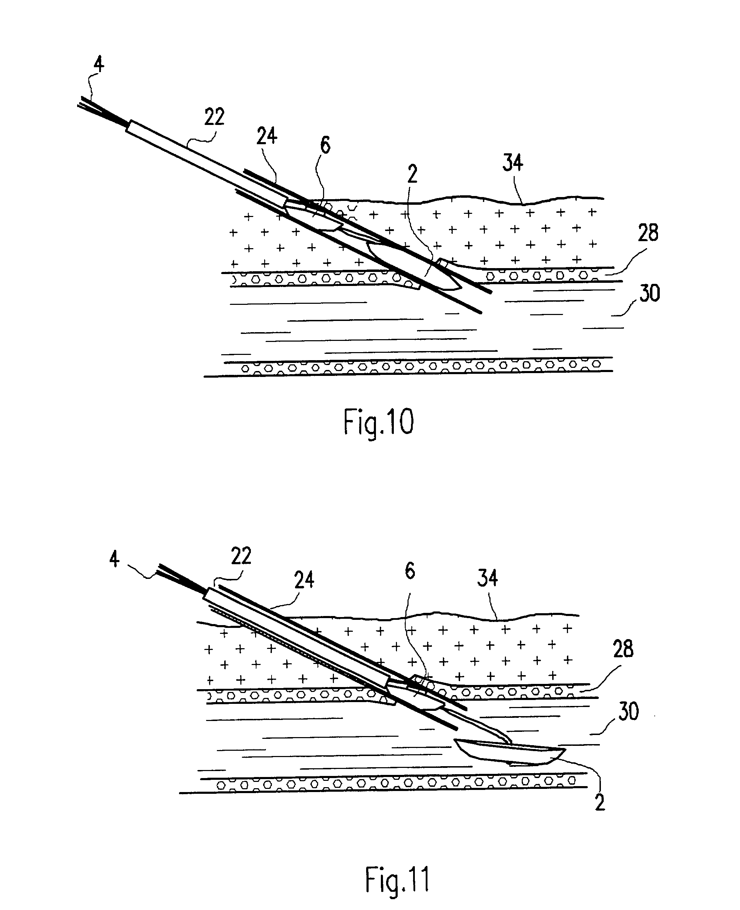

**Figure 10** shows a sectional view of the wound site, with the pusher 22, the first sealing member 2, the elongated member 4 and the second sealing member 6, inside of the introducer 24. As can be seen in **Figure 10,** the first sealing member 2 has been pushed such that it is situated in the distal end of the introducer 24. The first sealing member 2 is in a compressed state. Thereafter, the pusher 22 is pushed until a first sealing (not shown) of the pusher 22 abuts the proximal end of the introducer 24 and the first sealing member 2 has reached the state shown in **Figure 11**. In this state the first sealing member 2 extends outside of the introducer 24 and into the vessel 30. As soon as the first sealing member 2 is outside of the introducer 24 it will unfold to an expanded state.

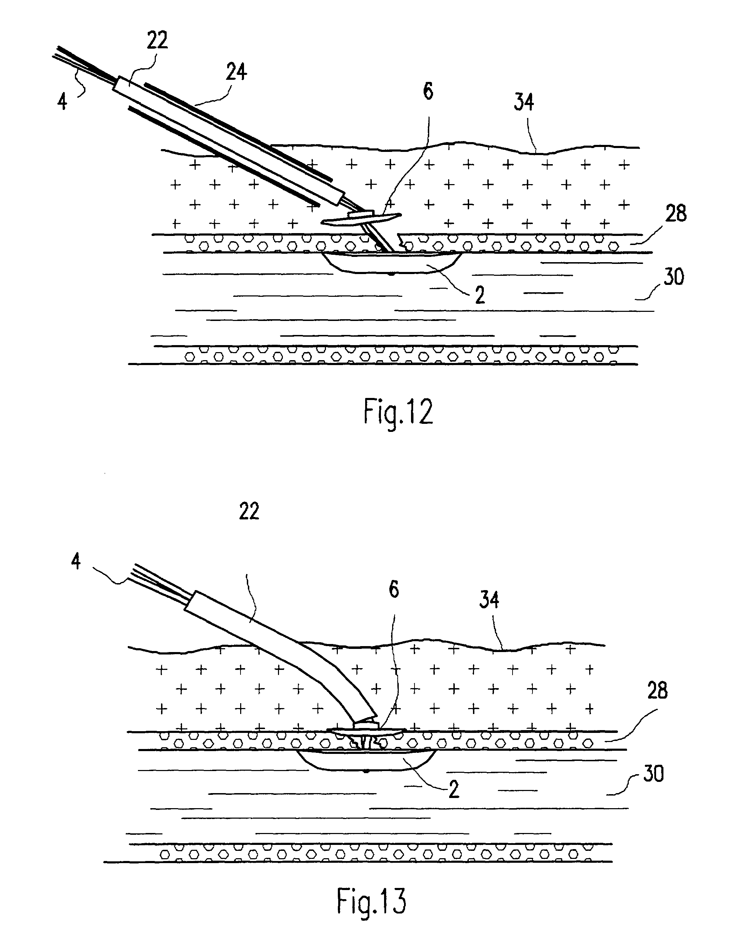

**Figure 12** shows the next state, wherein the introducer 24 is drawn out of the vessel 30 until it reaches the tissue outside of the vessel wall 28. The second sealing member 6 is pushed outside the introducer 24.

Thereafter the pusher 22 pushes the second sealing member 6 along the elongated member 4, over the distal lock portion 16, into its final position. **(Figure 13**) During the process of bringing the second sealing member 6, the elongate member 4 of the first sealing member 2 will act as a guide towards that position. The second sealing member 6 is threadable onto and along the elongate member 4. When the second sealing member 6 is close to its final position, the elongate member 4 onto which it is threaded increases in diameter. The second sealing member 6 will therefore be in frictional engagement with the elongated member 4 when it reaches its final position.

Thereafter the pusher 22 and the introducer 24 are removed and the sealing device closes the wound as can be seen in **Figure 14.**

Since the second sealing member 6 is threadable onto and along the elongated member 4, it is possible to adapt the sealing device to wounds having different thickness. One of the great advantages with the sealing device of the present invention is that it is adaptable to the thickness of the wound or vessel wall 28.

It shall be noted that the different features depicted in the figures are not drawn in scale. The purpose of the figures is not to limit the invention to the dimensions or relations shown, but instead make it easy to understand the principles of the present invention.

Whilst this invention has been described in terms of preferred embodiments thereof, it will be appreciated that other forms could readily be adapted by one skilled in the art.
Accordingly, the scope of this invention is to be considered limited only by the following claims and equivalents thereof.

## Claims

1. A sealing device for closing a wound in a wall of a vessel,
said sealing device comprising:
a first sealing member (2) attached to a distal end of an elongate member (4) and a second sealing member (6),
said second sealing member (6) comprising an opening (14) such that said sealing member (6) is threadable onto and along the elongate member (4) and,
said elongate member (4) comprises:
a proximal portion with a thickness smaller than said opening and
a distal lock portion (16);
**characterised in that**
said elongate member (4) is a suture wire and
said distal lock portion (16) has a constant thickness along said lock portion (16), said thickness being greater than said opening (14) of the second sealing member (6), so that when said second sealing member (6) is thread onto and along the elongate member (4), it is infinitely variable lockable along said distal lock portion(16).

2. Sealing device according to claim 1, wherein said suture comprises a hollow core, which hollow core includes means (18) for thickening the suture in the distal lock portion.

3. Sealing device according to claim 2, wherein the thickness of the distal portion of the elongate member (4) is adapted for frictional engagement to the sealing member (6).

4. Sealing device according to any of the previous claims, wherein the elongated member (4) is a pair of suture wires (12).

5. Sealing device according to any of the previous claims, wherein the second sealing member (6) is divided into two parts, a first part (41) and a second part (42), which first part (41) is a plate and is provided with an opening that is the same or greater than the thickness of the distal lock portion (16), and which the second part (42) is provided with an opening that is smaller than the thickness of the distal lock portion (16) and which second part (42) allows for frictional engagement between the inside of an opening of the second part (42) and the distal portion (16).

6. A sealing device for closing a wound in a wall of a vessel,
said sealing device comprising:
a first sealing member (2) attached to a distal end of an elongate member (4) and a second sealing member (6),
said sealing member (6) comprising an opening such that said second sealing member (6) is threadable onto and along the elongate member (4) and,
said elongate member (4) comprises:
a proximal portion with a thickness smaller than said opening (14) and
a distal lock portion (16);
**characterised in that**
said distal lock portion (16) is coated by a hollow stocking-like suture wire so that
said distal lock portion (16) has a constant thickness along the lock portion, said thickness being greater than said opening of the second sealing member (6), so that when said second sealing member (6) is thread onto and along the elongate member (4) it is infinitely variable lockable along said distal lock portion (16).

7. Wound closure device **characterised in that** it comprises a sealing device according to any of the claims 1-6 and a pusher (22) adapted for pushing, through an introducer (24), the first sealing member (2) and the elongated member (4) through a wound in a vessel wall and the second sealing member (6) towards the outside of said wound to frictional engagement to the elongated member (4) so as to seal the sealing device.

## Patentansprüche

1. Versiegelungsvorrichtung zum Verschließen einer Verletzung in der Wand eines Gefäßes, die Versiegelungsvorrichtung weist auf:
ein erstes Versiegelungselement (2), das an dem distalen Ende eines langgestreckten Elementes (4) befestigt ist, und ein zweites Versiegelungselement (6), welches zweite Versiegelungselement (6) eine solche Öffnung (14) aufweist, dass das Versiegelungselement (6) auf und entlang des langgestreckten Elementes (4) fädelbar ist, und das langgestreckte Element weist auf:
einen proximalen Abschnitt mit einer geringeren Dicke als die Öffnung und einen distalen Verriegelungsabschnitt (16);
**dadurch gekennzeichnet, dass**
das langgestreckte Element (4) ein Wundfaden ist und
der distale Verriegelungsabschnitt (16) eine konstante Dicke entlang des distalen Verriegelungsabschnitts (16) besitzt, welche Dicke größer ist als die Öffnung (14) des zweiten Versiegelungselementes (6), so dass es, wenn das zweite Versiegelungselement auf und entlang des langgestreckten Elementes (4) gefädelt ist, unendlich variabel verriegelbar entlang des distalen Verriegelungsabschnitts (16) ist.

2. Versiegelungsvorrichtung nach Anspruch 1, worin der Wundfaden einen hohlen Kern besitzt, welcher hohle Kern Einrichtungen (18) zum Verdicken des Wundfadens in dem distalen Verriegelungsabschnitt einschließt.

3. Versiegelungsvorrichtung nach Anspruch 2, worin die Dicke des distalen Abschnitts des langgestreckten Elementes (4) für Reibschluss mit dem Versiegelungselement (6) angepasst ist.

4. Versiegelungsvorrichtung nach einem der vorangehenden Ansprüche, worin das langgestreckte Element (4) ein Paar Wundfäden (1, 2) ist.

5. Versiegelungsvorrichtung nach einem der vorangehenden Ansprüche, worin das zweite Versiegelungselement (6) in zwei Teile aufgeteilt ist, einen ersten Teil (41) und einen zweiten Teil (42), welcher erste Teil ein Platte ist und mit einer Öffnung ausgestattet ist, die genauso groß oder größer ist als die Dicke des distalen Verriegelungsabschnitts (16), und welcher zweite Teil (4) mit einer Öffnung ausgestattet ist, die kleiner ist als die Dicke des distalen Verriegelungsabschnitts (16) und welcher zweite Teil (42) einen Reibschluss zwischen dem Inneren einer Öffnung des zweiten Teils (42) und dem distalen Abschnitt (16) ermöglicht.

6. Versiegelungsvorrichtung zum Verschließen einer Verletzung in der Wand eines Gefäßes, die Versiegelungsvorrichtung weist auf:
ein erstes Versiegelungselement (2), das an dem distalen Ende eines langgestreckten Elementes (4) befestigt ist, und ein zweites Versiegelungselement (6), welches zweite Versiegelungselement (6) eine solche Öffnung (14) aufweist, dass das Versiegelungselement (6) auf und entlang des langgestreckten Elementes (4) fädelbar ist, und das langgestreckte Element weist auf:
einen proximalen Abschnitt mit einer geringeren Dicke als die Öffnung und einen distalen Verriegelungsabschnitt (16);
**dadurch gekennzeichnet, dass**
der distale Verriegelungsabschnitt (16) mit einem hohlen, strumpf-artigem Wundfaden ummantelt ist, so dass der distale Verriegelungsabschnitt (16) entlang des Verriegelungsabschnitts eine konstante Dicke besitzt, welche Dicke größer als die Öffnung des zweiten Versiegelungselementes (6) ist, so dass es, wenn das zweite Versiegelungselement auf und entlang des langgestreckten Elementes (4) gefädelt ist, unendlich variabel verriegelbar entlang des distalen Verriegelungsabschnitts (16) ist.

7. Wundverschlussvorrichtung, **dadurch gekennzeichnet, dass** es eine Versiegelungsvorrichtung nach einem der Ansprüche 1 bis 6 und einen Schieber (22) aufweist, der dazu ausgelegt ist, durch einen Einführer (24) hindurch, das erste Versiegelungselement (2) und das langgestreckte Element (4) durch eine Verletzung in einer Gefäßwand und das zweite Versiegelungselement (6) zu dem äußeren der Verletzung hin in Reibschluss mit dem langgestreckten Element (4) zu schieben, um das Versiegelungselement zu versiegeln.

## Revendications

1. Dispositif d'obturation pour fermer une plaie dans une paroi d'un vaisseau,
ledit dispositif d'obturation comportant :
un premier élément d'obturation (2) attaché à une extrémité distale d'un élément allongé (4) et un second élément d'obturation (6),
ledit second élément d'obturation (6) présentant une ouverture (14) de façon que ledit élément d'obturation (6) puisse être enfilé sur et le long de l'élément allongé (4) et
ledit élément allongé (4) comporte :
une partie proximale ayant une épaisseur inférieure à celle de ladite ouverture et
une partie distale (16) de verrouillage ;
**caractérisé en ce que**
ledit élément allongé (4) est un fil à suture et
ladite partie distale de verrouillage (16) a une épaisseur constante le long de ladite partie de verrouillage (16), ladite épaisseur étant supérieure à celle de ladite ouverture (14) du second élément d'obturation (6), afin que, lorsque ledit second élément d'obturation (6) est enfilé sur et le long de l'élément allongé (4), il puisse être verrouillé d'une manière variant à l'infini le long de ladite partie distale (16) de verrouillage.

2. Dispositif d'obturation selon la revendication 1, dans lequel ladite suture comporte une âme creuse, laquelle âme creuse comprend un moyen (18) destiné à épaissir la suture dans la partie distale de verrouillage.

3. Dispositif d'obturation selon la revendication 2, dans lequel l'épaisseur de la partie distale de l'élément allongé (4) est adaptée pour réaliser un engagement à frottement avec l'élément d'obturation (6).

4. Dispositif d'obturation selon l'une quelconque des revendications précédentes, dans lequel l'élément allongé (4) est une paire de fils à suture (12).

5. Dispositif d'obturation selon l'une quelconque des revendications précédentes, dans lequel le second élément d'obturation (6) est divisé en deux parties, une première partie (41) et une seconde partie (42), laquelle première partie (41) est une plaque et est pourvue d'une ouverture qui est identique ou supérieure à l'épaisseur de la partie distale (16) de verrouillage, et laquelle seconde partie (42) est pourvue d'une ouverture qui est inférieure à l'épaisseur de la partie distale (16) de verrouillage, et laquelle seconde partie (42) permet un engagement à frottement entre l'intérieur d'une ouverture de la seconde partie (42) et la partie distale (16).

6. Dispositif d'obturation destiné à fermer une plaie dans une paroi d'un vaisseau,
ledit dispositif d'obturation comportant :
un premier élément d'obturation (2) attaché à une extrémité distale d'un élément allongé (4) et un second élément d'obturation (6),
ledit élément d'obturation (6) présentant une ouverture telle que ledit second élément d'obturation (6) peut être enfilé sur et le long de l'élément allongé (4), et
ledit élément allongé (4) comporte :
une partie proximale ayant une épaisseur inférieure à celle de ladite ouverture (14) et
une partie distale (16) de verrouillage ;
**caractérisé en ce que**
ladite partie distale (16) de verrouillage est revêtue d'un fil à suture analogue à un bas creux afin que ladite partie distale (16) de verrouillage ait une épaisseur constante le long de la partie de verrouillage, ladite épaisseur étant supérieure à celle de ladite ouverture du second élément (6) d'obturation, afin que, lorsque ledit second élément (6) d'obturation est enfilé sur et le long de l'élément allongé (4), il puisse être verrouillé d'une façon variant à l'infini le long de ladite partie distale (16) de verrouillage.

7. Dispositif pour fermer une plaie, **caractérisé en ce qu'**il comporte un dispositif d'obturation selon l'une quelconque des revendications 1 à 6 et un poussoir (22) conçu pour pousser, à travers un élément d'introduction (24), le premier élément d'obturation (2) et l'élément allongé (4) à travers une plaie dans une paroi d'un vaisseau et le second élément d'obturation (6) vers le côté extérieur de ladite plaie afin de réaliser un engagement à frottement avec l'élément allongé (4) pour obturer le dispositif d'obturation.
